# EUROPEAN PATENT SPECIFICATION

(11) **EP 1 706 177 B1**
(45) Date of publication and mention of the grant of the patent: **19.08.2009**
(21) Application number: 04815705.1
(22) Date of filing: 23.12.2004
(51) Int. Cl.: A61N 1/36, A61N 1/05

(54) **LEAD FOR STIMULATING THE BARORECEPTORS IN THE PULMONARY ARTERY**
LEITUNG ZUR STIMULIERUNG DER BAROREZEPTOREN IN DER PULMONALARTERIE
CORDON PERMETTANT DE STIMULER LES BARORECEPTEURS SITUE DANS L'ARTERE PULMONAIRE

(30) Priority: 24.12.2003 US 746852; 24.12.2003 US 746861
(43) Date of publication of application: 04.10.2006
(73) Proprietor: CARDIAC PACEMAKERS, INC., St. Paul, Minnesota 55112 (US)
(72) Inventor: LIBBUS, Imad, St. Paul, MN 55105 (US); HEIL, Ronald, W., Jr., Roseville, MN 55113 (US); SCHEINER, Avram, Vadnais Heights, MN 55127 (US); KELLEY, Peter, T., Buffalo, MN 55313 (US)
(74) Representative: Vossius & Partner
(86) International application number: PCT/US2004/043691
(87) International publication number: WO 2005/065771

(56) References cited:
- WO-A-03/082080
- US-A1- 2002 120 304
- US-B1- 6 178 349
- US-B1- 6 522 926

## Description

### Field

This application relates generally to implantable medical devices and, more particularly, to systems, devices for reducing hypertension using baroreceptor stimulation.

### Background

Medical leads have a distal end having one or more electrodes and a proximal end having a terminal that is coupled to a pulse generator. Electrical therapy is delivered from the pulse generator to the body via the electrode.

Hypertension is a cause of heart disease and other related cardiac co-morbidities. Hypertension occurs when blood vessels constrict. As a result, the heart works harder to maintain flow at a higher blood pressure, which can contribute to heart failure. Many patients who suffer from hypertension do not respond to treatment, such as treatments related to lifestyle changes and hypertension drugs.

A pressoreceptive region is capable of sensing changes in pressure, such as changes in blood pressure. Pressoreceptor regions are referred to herein as baroreceptors, which generally include any sensors of pressure changes. For example, baroreceptors include afferent nerves and further include sensory nerve endings that are sensitive to the stretching of the wall that results from increased blood pressure from within, and function as the receptor of a central reflex mechanism that tends to reduce the pressure. Baroreflex functions as a negative feedback system, and relates to a reflex mechanism triggered by stimulation of a baroreceptor. Increased pressure stretches blood vessels, which in turn activate baroreceptors in the vessel walls. Activation of baroreceptors naturally occurs through internal pressure and stretching of the arterial wall, causing baroreflex inhibition of sympathetic nerve activity (SNA) and a reduction in systemic arterial pressure. An increase in baroreceptor activity induces a reduction of SNA, which reduces blood pressure by decreasing peripheral vascular resistance.

The general concept of stimulating afferent nerve trunks leading from baroreceptors is known. For example, direct electrical stimulation has been applied to the vagal nerve and carotid sinus using nerve cuffs. Research has indicated that electrical stimulation of the carotid sinus nerve can result in reduction of experimental hypertension, and that direct electrical stimulation to the pressoreceptive regions of the carotid sinus itself brings about reflex reduction in experimental hypertension.

What is needed is a less invasive technique for providing long-term electrical stimulation of the baroreflex.
Document US-B-6 522 926 represents the most relevant prior art.

### Summary

One aspect includes a flexible lead body extending from a proximal end to a distal end and having an expandable electrode coupled proximate the distal end of the lead body. The expandable electrode has an expanded diameter dimensioned to abut a wall of a pulmonary artery. An implantable pulse generator is electrically coupled to the expandable electrode and is adapted to deliver a baroreflex stimulation signal to a baroreceptor in the pulmonary artery via the expandable electrode.

One aspect provides a lead for placement in the pulmonary artery. The lead includes a flexible lead body extending from a proximal end to a distal end, the distal end having a biased portion having an outer diameter dimensioned to abut a wall of a pulmonary artery, an electrode coupled proximate the distal end. An implantable pulse generator is electrically coupled to the electrode. The implantable pulse generator is adapted to deliver a baroreflex stimulation signal to a baroreceptor in the pulmonary artery via the electrode.

The invention is defined in claims 1, 8, 19.

### Brief Description of the Drawings

Figure 1 shows a lead and pulse generator, in accordance with one embodiment.
Figure 2 shows details of a heart.
Figure 3 shows the lead of Figure 1 implanted in a heart, in accordance with one embodiment.
Figure 4 shows a lead having an expandable electrode in accordance with one embodiment.
Figure 5 shows the lead of Figure 4 with the electrode in an expanded configuration.
Figure 6 shows an expandable electrode in accordance with one embodiment.
Figure 7 shows a side view of a lead in accordance with one embodiment.
Figure 8 shows a lead and pulse generator, in accordance with one embodiment.
Figure 9 shows the lead of Figure 8 implanted in a heart, in accordance with one embodiment.
Figure 10 shows a lead in accordance with one embodiment.
Figure 11 shows the lead of Figure 10 with the distal end in a biased configuration.
Figure 12 shows a lead in accordance with one embodiment.
Figure 13 shows a side view of a lead in accordance with one embodiment.
Figure 14 shows a portion of a lead in accordance with one embodiment.
Figure 15 shows a lead implanted in accordance with one embodiment.

### Detailed Description

The following detailed description and accompanying drawings show specific embodiments in which the present invention may be practiced. These embodiments are described in sufficient detail to enable those skilled in the art to practice the invention. Other embodiments may be utilized and structural changes may be made without departing from the scope of the present invention.

A brief discussion of hypertension and the physiology related to baroreceptors is provided to assist the reader with understanding this disclosure. This brief discussion introduces hypertension, the autonomic nervous system, and baroreflex.

Hypertension is a cause of heart disease and other related cardiac co-morbidities. Hypertension generally relates to high blood pressure, such as a transitory or sustained elevation of systemic arterial blood pressure to a level that is likely to induce cardiovascular damage or other adverse consequences. Hypertension has been arbitrarily defined as a systolic blood pressure above 140 mm Hg or a diastolic blood pressure above 90 mm Hg. Hypertension occurs when blood vessels constrict. As a result, the heart works harder to maintain flow at a higher blood pressure. Consequences of uncontrolled hypertension include, but are not limited to, retinal vascular disease and stroke, left ventricular hypertrophy and failure, myocardial infarction, dissecting aneurysm, and renovascular disease.

The automatic nervous system (ANS) regulates "involuntary" organs, while the contraction of voluntary (skeletal) muscles is controlled by somatic motor nerves. Examples of involuntary organs include respiratory and digestive organs, and also include blood vessels and the heart. Often, the ANS functions in an involuntary, reflexive manner to regulate glands, to regulate muscles in the skin, eye, stomach, intestines and bladder, and to regulate cardiac muscle and the muscle around blood vessels, for example.

The ANS includes, but is not limited to, the sympathetic nervous system and the parasympathetic nervous system. The sympathetic nervous system is affiliated with stress and the "fight or flight response" to emergencies. Among other effects, the "fight or flight response" increases blood pressure and heart rate to increase skeletal muscle blood flow, and decreases digestion to provide the energy for "fighting or fleeing." The parasympathetic nervous system is affiliated with relaxation and the "rest and digest response" which, among other effects, decreases blood pressure and heart rate, and increases digestion to conserve energy. The ANS maintains normal internal function and works with the somatic nervous system.

The subject matter of this disclosure generally refers to the effects that the ANS has on the heart rate and blood pressure, including vasodilation and vasoconstriction. The heart rate and force is increased when the sympathetic nervous system is stimulated, and is decreased when the sympathetic nervous system is inhibited (e.g. when the parasympathetic nervous system is stimulated).

Baroreflex is a reflex triggered by stimulation of a baroreceptor. A baroreceptor includes any sensor of pressure changes, such as sensory nerve endings in the wall of the auricles of the heart, cardiac fat pads, vena cava, aortic arch and carotid sinus, that is sensitive to stretching of the wall resulting from increased pressure from within, and that functions as the receptor of the central reflex mechanism that tends to reduce that pressure. Additionally, a baroreceptor includes afferent nerve trunks, such as the vagus, aortic and carotid nerves, leading from the sensory nerve endings. Stimulating baroreceptors inhibits sympathetic nerve activity (stimulates the parasympathetic nervous system) and reduces systemic arterial pressure by decreasing peripheral vascular resistance. Baroreceptors are naturally stimulated by internal pressure and the stretching of the arterial wall.

Some aspects of the present subject matter locally and directly stimulate specific nerve endings in arterial walls rather than stimulate afferent nerve trunks in an effort to stimulate a desire response (e.g. reduced hypertension) while reducing the undesired effects of indiscriminate stimulation of the nervous system. For example, some embodiments stimulate baroreceptor sites in the pulmonary artery. Some embodiments of the present subject matter involve stimulating baroreceptor sites in the aorta.

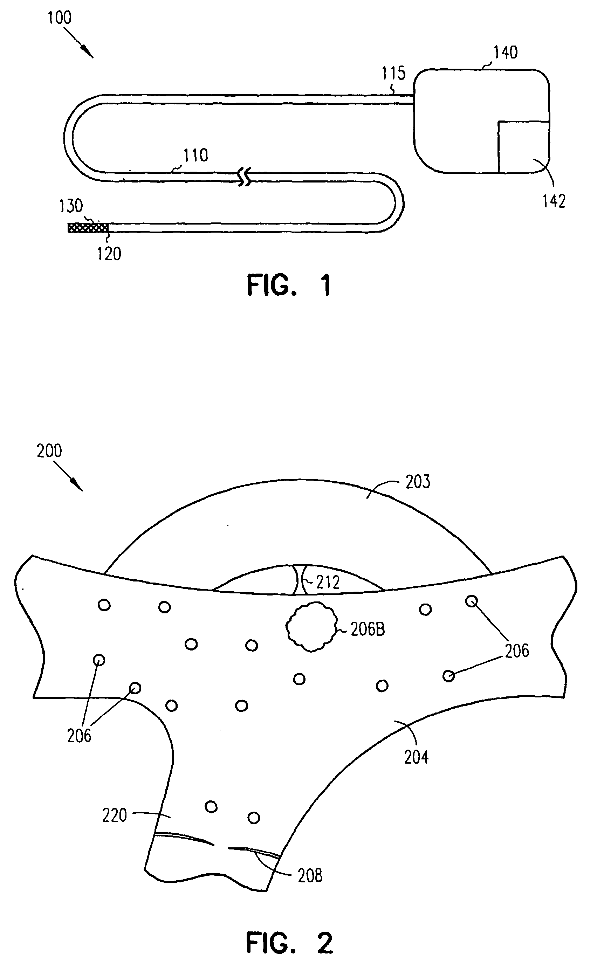

Figure 1 shows a lead 100 according to one embodiment. Lead 100 includes a flexible lead body 110 extending from a proximal end 115 to a distal end 120. An expandable electrode 130 is coupled proximate the distal end 120 of lead body 110. As will be discussed below, electrode 130 is adapted to deliver stimulation to baroreceptors in the pulmonary artery. For example, the expandable electrode 130 can have an expanded diameter dimensioned to abut a wall of a pulmonary artery to hold the electrode in place without any active fixation.

Lead 100 is coupled to an implantable pulse generator 140. Lead 100 includes conductors, such as coiled conductors that electrically couple pulse generator 140 to expandable electrode 130. Accordingly, implantable pulse generator 140 can deliver a baroreflex stimulation signal to a baroreceptor in the pulmonary artery via the electrode 130. The lead further includes outer insulation to insulate the conductor. The system can include a unipolar system with the case acting as an electrode or a bipolar system with a pulse between two distally located electrodes.

In one embodiment, pulse generator 140 includes hardware, circuitry and software to perform NS functions. Some embodiments can also perform an AHT function. Pulse generator includes controller circuitry 142. The controller circuitry 142 is capable of being implemented using hardware, software, and combinations of hardware and software. For example, according to various embodiments, the controller circuitry 142 includes a processor to perform instructions embedded in a memory to perform functions associated with NS therapy such as AHT therapy. In one example, the pulse generator delivers a pulse train having a frequency of between 10 to 150 hertz via the electrode.

Figure 2 shows a representation of a portion of a heart 200. Heart 200 includes an aortic arch 203 and a pulmonary artery 204. Pulmonary artery 204 includes a plurality of baroreceptors 206. A lead, such as lead 100 (Fig. 1), is capable of being intravascularly inserted through a peripheral vein and through the tricuspid valve into the right ventricle of the heart (not expressly shown in the figure) similar to a cardiac pacemaker lead, and continue from the right ventricle through the pulmonary valve 208 into the pulmonary artery 204.

A portion of the pulmonary artery 204 and aorta arch 203 are proximate to each other. According to various aspects of the present subject matter, the baroreflex is stimulated in or around the pulmonary artery by at least one electrode intravascularly inserted into the pulmonary artery. Aspects of the present subject matter provide a relatively noninvasive surgical technique to implant a baroreceptor stimulator intravascularly into the pulmonary artery. The baroreceptors 206, for example, are sensitive to stretching of the wall resulting from increased pressure from within. Activation of these nerve endings reduce pressure.

As illustrated, the pulmonary artery 204 includes a number of baroreceptors 206, as generally indicated by the dark areas. Furthermore, a cluster of closely spaced baroreceptors 206B are situated near the attachment of the ligamentum arteriosum 212. According to various embodiments of the present subject matter, a lead is inserted through a peripheral vein and threaded through the tricuspid valve into the right ventricle, and from the right ventricle through the pulmonary valve 208 and into the pulmonary artery 204 to simulate baroreceptors in and/or around the pulmonary artery. In various embodiments, for example, the lead is positioned in the artery to stimulate the cluster of baroreceptors 206B near the ligamentum arteriosum 212.

There are also baroreceptor fields in the aortic arch 203, near the ligamentum arteriosum 212, and in the trunk 220 of the pulmonary artery 204. Some embodiments position the lead in the pulmonary artery to stimulate baroreceptor sites in the aorta, possibly via the ligamentum arteriosum 212, or in the pulmonary trunk, or in either the right or left pulmonary arteries.

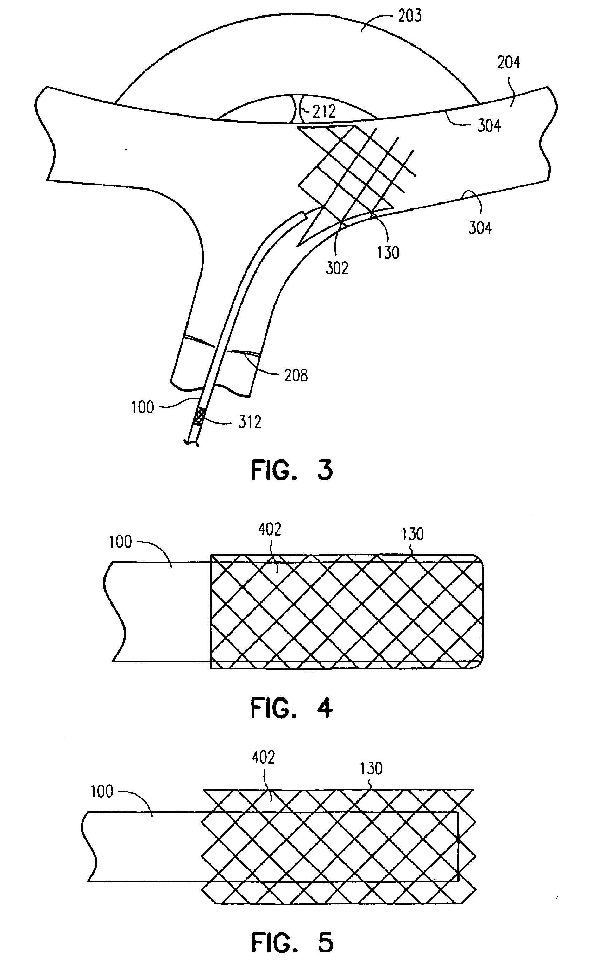

Figure 3 shows expandable electrode 130 mounted in pulmonary artery 204, in accordance with one embodiment. In one embodiment, expandable electrode 130 is a stent-like structure including a mesh surface 302. The expandable electrode is designed to expand to abut the outer walls 304 of the pulmonary artery so as to passively fixate the lead and the electrode in place by frictional forces. In this example, the electrode is adapted to be located near the ligamentum arteriosum 212 of the left pulmonary artery 204.

Expandable electrode 130 can have various shapes and sizes. In one embodiment, the length to diameter ratio is smaller than in typical stents. For example, one embodiment includes a length of at least about 1 cm. Other examples can be up to 3 cm or greater. The diameter of the electrode 130 in its expanded orientation can range from about 10 mm to about 20 mm. Other embodiments can have a larger diameter. In general, the electrode is dimensioned to provide direct, low-voltage, high-frequency nerve stimulation.

In one embodiment, lead 100 can include a second electrode 312 located proximally from the expandable electrode 130. This electrode can be used for bradyarrhythmia therapy, tachyarrhythmia therapy, as a sensing electrode, or as a cathode for electrode 130.

In one example, electrode 130 is adapted to be chronically implanted in the pulmonary artery. For example, by passively mounting the electrode within the artery, no long-term damage is done to the artery.

Figures 4 and 5 show lead 100, in accordance with one embodiment. Lead 100 can include an inflatable balloon 402 that can be inflated once the electrode is positioned correctly. Inflating the balloon 402 expands the electrode 130 until the electrode abuts the walls of the artery, as discussed above.

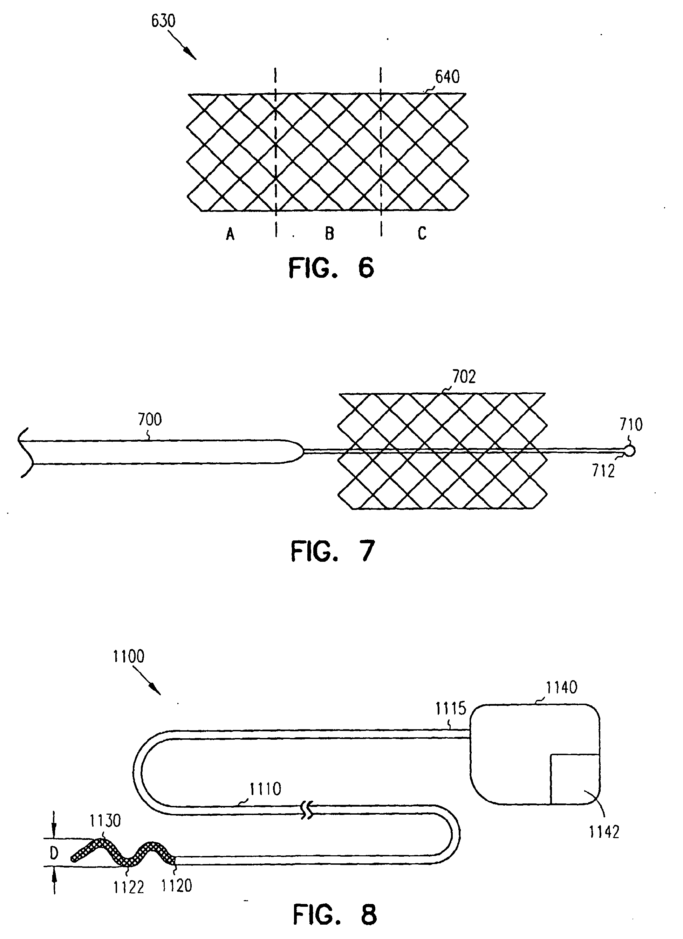

Figure 6 shows an expandable electrode 630, in accordance with one embodiment. Electrode 630 can be used in place of electrode 130 discussed above. Electrode 630 includes an outer surface 640 that can be at least partially masked so as to be electrically non-conductive. For example, electrode 630 has been marked off into zones A, B, and C. Zones A, B, and C can have varying sizes and shapes and the zones shown are an example. In one example, sections A and C can be electrically conductive and zone B is masked off. Alternatively, any of the sections or portions can be electrically insulated.

In one embodiment, none of sections A, B, or C is masked off and each is coupled to a separate conductor of lead 100 (Figure 1). In this example, the user implants the electrode and then tests each zone separately to discover which gives the best baroreflex response. Then, the non-productive zones can be turned off, or used for sensing, for example.

Figure 7 illustrates a baroreceptor stimulation lead 700 with an integrated pressure sensor 710 (IPS) and an expandable electrode 702, according to various embodiments of the present subject matter. In one example, lead 700 can include an IPS 710 with a baroreceptor stimulator electrode 712 to monitor changes in blood pressure, and thus to monitor the effect of the baroreceptor stimulation. In various embodiments, micro-electrical mechanical systems (MEMS) technology can be used to sense the blood pressure. Some sensor embodiments determine blood pressure based on a displacement of a membrane.

In one example use of lead 700, a system can include baroreceptor stimulation circuitry and sensor circuitry. The circuitry can be within pulse generator 140 (Figure 1) or be a separate system communicating wirelessly with the pulse generator. One or more leads can be connected to the sensor circuitry and baroreceptor stimulation circuitry. The baroreceptor stimulation circuitry is used to apply electrical stimulation pulses to desired baroreceptors sites, such as baroreceptor sites in the pulmonary artery, through one or more stimulation electrodes, such as electrodes 702 or 712. The sensor circuitry is used to detect and process ANS nerve activity and/or surrogate parameters such as blood pressure, respiration and the like, to determine the ANS activity.

Lead 700, for example, is capable of being intravascularly introduced to stimulate a baroreceptor site, such as the baroreceptor sites in the pulmonary artery, aortic arch, or ligamentum arteriosum. One or more additional electrodes can be provided to pace and/or sense cardiac activity, such as that which may occur within the right ventricle with the sensor 710 located near baroreceptors in or near the pulmonary artery and programmed to stimulate and sense, either directly or indirectly through surrogate parameters, baroreflex activity.

Figure 8 shows a lead 1100 according to one embodiment. Lead 1100 includes a flexible lead body 1110 extending from a proximal end 1115 to a distal end 1120. An electrode 1130 is coupled proximate the distal end 1120 of lead body 1110. The distal end 1120 includes a biased portion 1122. Lead 1100 is adapted to deliver stimulation to baroreceptors in the pulmonary artery. For example, the biased portion 1122 can have a biased configuration having an outer diameter, D, dimensioned to abut a wall of a pulmonary artery to hold the electrode in place without any active fixation, as will be explained below.

Lead 1100 is coupled to an implantable pulse generator 1140. Lead 1100 includes conductors, such as coiled conductors that electrically couple pulse generator 1140 to electrode 1130. Accordingly, implantable pulse generator 1140 can deliver a baroreflex stimulation signal to a baroreceptor in the pulmonary artery via the electrode 1130. The lead further includes outer insulation to insulate the conductor. The system can include a unipolar system with the case acting as an electrode or a bipolar system with a pulse between two distally located electrodes.

In one embodiment, pulse generator 1140 includes hardware, circuitry and software to perform NS functions. Some embodiments can also perform an AHT function. Pulse generator includes controller circuitry 1142. The controller circuitry 1142 is capable of being implemented using hardware, software, and combinations of hardware and software. For example, according to various embodiments, the controller circuitry 1142 includes a processor to perform instructions embedded in a memory to perform functions associated with NS therapy such as AHT therapy. In one example, the pulse generator delivers a pulse train having a frequency of between about 10 to 150 hertz via the electrode. In one embodiment, the pulse generator can deliver a waveform of 30 hertz, 2.5 msec, at 5 volts. Some embodiments deliver a waveform of 100 hertz, 1.0 msec, at 1, 3, or 5 volts, for example.

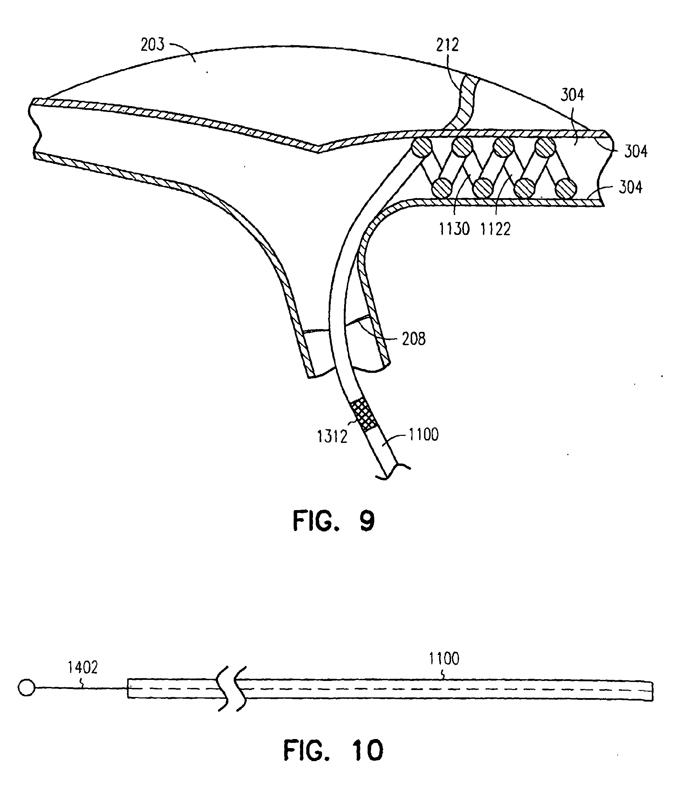

Figure 9 shows a cross-section of electrode 1130 mounted in pulmonary artery 204, in accordance with one embodiment. In one embodiment, biased portion 1122 includes an elongated spiral configuration with electrode 1130 being a coiled electrode that is exposed to have substantially complete annular contact with the wall surface 304 of the pulmonary artery 204. This allows for distributed stimulation along the length of the biased portion 1122 and distributed wall fixation. Thus, for example, the entire electrode 1130 can be an exposed electrode manufactured in a spiral configuration. As will be discussed below, some embodiments can include two or more discrete electrodes placed along the distal end of the lead. In some embodiments, portions of the surface of the electrode can be masked off to be electrically insulated.

As noted, the outer diameter of the biased portion 1122 is designed to expand to abut the outer walls 304 of the pulmonary artery so as to fix the lead and the electrode in place by frictional forces. In this example, the electrode is adapted to be located near the ligamentum arteriosum 212 of the left pulmonary artery 204. In some embodiments, at least a portion of electrode 1130 can include a porous surface to further help fix the electrode within the artery.

In one embodiment, the biased portion 1122 can have an outer biased diameter of about 10 mm to about 20 mm. Other embodiments can have a larger diameter. One example has a pitch of at least 5 coils per inch to provide high arterial surface contact and increased fixation. In general, the distal end and the electrode are dimensioned to optimize direct, low-voltage, high-frequency nerve stimulation.

In one embodiment, lead 1100 can include a second electrode 1312 located proximally from electrode 1130. This electrode can be used for bradyarrhythmia therapy, tachyarrhythmia therapy, as a sensing electrode, or as a cathode for electrode 1130.

In one example, electrode 1130 is adapted to be chronically implanted in the pulmonary artery. For example, by passively mounting the electrode within the artery, no long-term damage is done to the artery.

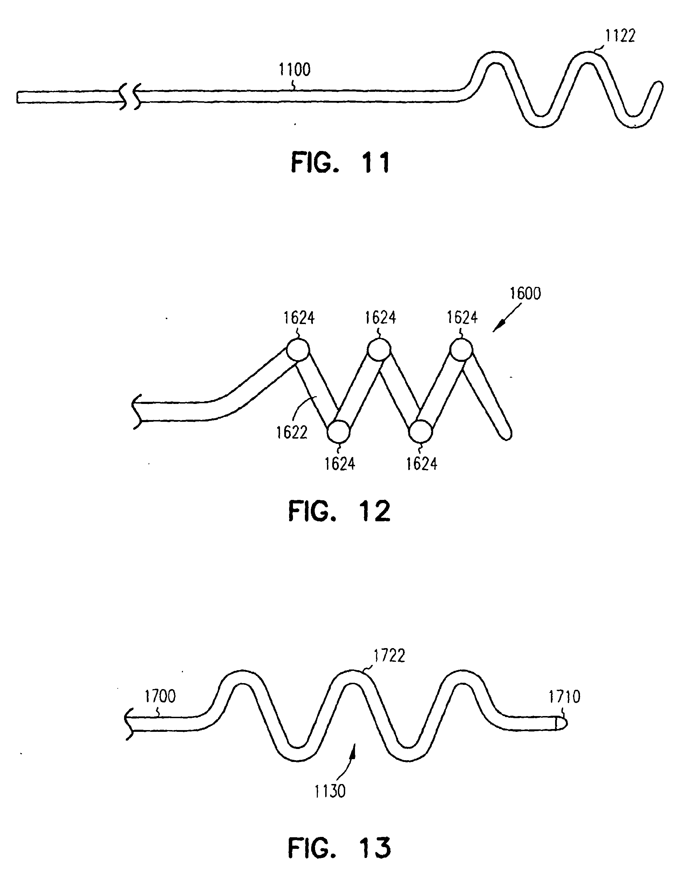

Figures 10 and 11 show lead 1100, in accordance with one embodiment. Lead 1100 can be implanted using a stylet 1402 that is removed once the lead is positioned correctly. Removing stylet 1402 from the lead allows distal end 1122 to expand to its biased configuration until the electrode abuts the walls of the artery, as discussed above.

Figure 12 shows a portion of a lead 1600, in accordance with one embodiment. Lead 1600 includes a biased distal portion 1622, similar to biased portion 1122 discussed above. In this embodiment, the biased portion 1622 includes two or more discrete electrodes 1624 exposed on the outer surface and outer diameter of the biased portion. In one embodiment, these discrete electrodes allow localized, but distributed stimulation when implanted in an artery. Alternatively, some of the discrete electrodes 1624 can be coupled to a separate conductor of lead 1600. In this example, the user implants the electrode and then tests each electrode separately to discover which gives the best baroreflex response. Then, the non-productive electrode or electrodes can be turned off, or used only for sensing, or other purposes. In some embodiments, discrete electrodes 1624 can be porous to provide further fixation within the artery.

Figure 13 illustrates a baroreceptor stimulation lead 1700 with an integrated pressure sensor 1710 (IPS), according to various embodiments of the present subject matter. Lead 1700 can include an IPS 1710 with a baroreceptor stimulator electrode to monitor changes in blood pressure, and thus to monitor the effect of the baroreceptor stimulation. In various embodiments, micro-electrical mechanical systems (MEMS) technology can be used to sense the blood pressure. Some sensor embodiments determine blood pressure based on a displacement of a membrane.

In one example use of lead 1700, a system can include baroreceptor stimulation circuitry and sensor circuitry. The circuitry can be within pulse generator 1140 (Figure 8) or be a separate system communicating wirelessly with the pulse generator. One or more leads can be connected to the sensor circuitry and baroreceptor stimulation circuitry. The baroreceptor stimulation circuitry is used to apply electrical stimulation pulses to desired baroreceptors sites, such as baroreceptor sites in the pulmonary artery, through one or more stimulation electrodes, such as electrode 1130 on biased portion 1722. The sensor circuitry is used to detect and process ANS nerve activity and/or surrogate parameters such as blood pressure, respiration and the like, to determine the ANS activity.

Lead 1700, for example, is capable of being intravascularly introduced to stimulate a baroreceptor site, such as the baroreceptor sites in the pulmonary artery, aortic arch, or ligamentum arteriosum. One or more additional electrodes can be provided to pace and/or sense cardiac activity, such as that which may occur within the right ventricle with the sensor 1710 located in or near the pulmonary artery and programmed to stimulate and sense, either directly or indirectly through surrogate parameters, baroreflex activity.

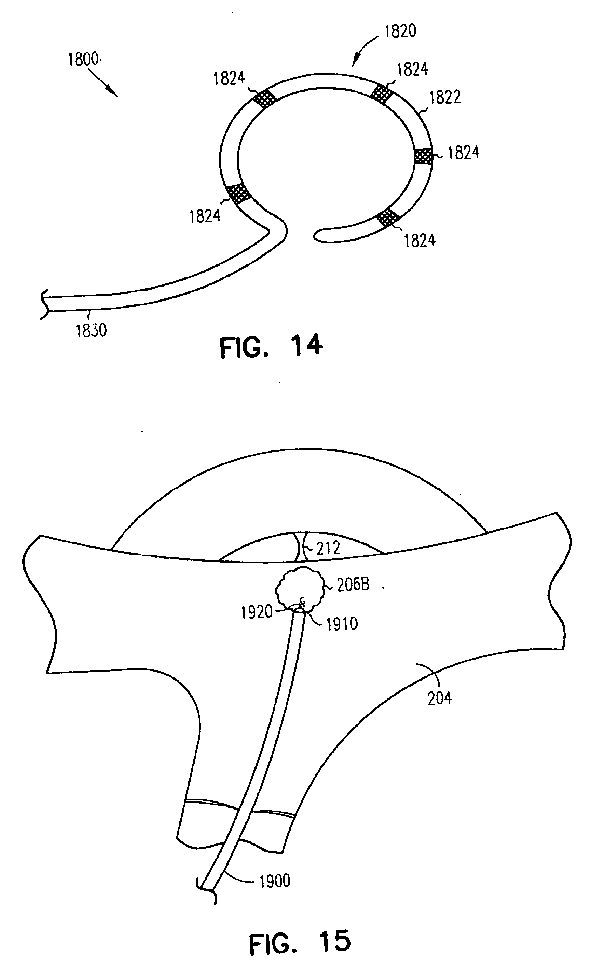

Figure 14 shows a lead 1800 according to one embodiment, lead 1800 includes a distal portion 1820 including a biased circular portion 1822. In this embodiment, a plurality of discrete electrodes 1824 are placed around the outer circumference of the circular portion 1822. In other embodiments, the entire biased circular portion can be an exposed electrode. Circular biased portion 1822 is dimensioned to fixate the distal end 1820 within the pulmonary artery. For example, the biased circular portion can have an outer diameter of between 10 mm and 20 mm. In one embodiment, the circular plane of the distal portion 1820 of the lead 1800 is perpendicular to the long axis 1830 of the lead body (i.e. shaped like a lasso). A distal end fashioned in this way tends to stimulate the pulmonary artery in a plane or cross section of the artery. Other embodiments can use other angles as needed.

To provide hypertension therapy according to one or more embodiments, a lead, such any lead discussed herein, is intravascularly inserted through a peripheral vein and through the tricuspid valve into the right ventricle of the heart and then from the right ventricle through the pulmonary valve into the pulmonary artery. An electrode on the lead is fixated to a region of the pulmonary artery having one or more baroreceptors. One example passively fixates the electrode proximate the ligamentum arteriosum of the left pulmonary artery. In one embodiment, the pulse generator is designed to intermittently pace the baroreceptor with a low-voltage, high frequency pulse train. For example, the baroreceptor can be paced for about 5 to 10 seconds each minute at a voltage of about 0.1 volts to 10 volts and a frequency between about 10 Hz and 150 Hz. Some examples utilize a voltage between about 1 volt to about 10 volts. One embodiment delivers at least a 10 hertz pulse train via the electrode. In some embodiments, the pulse generator can deliver a waveform of 30 hertz, 2.5 msec, at 5 volts. Some embodiments deliver a waveform of 100 hertz, 1.0 msec, at 1, 3, or 5 volts, for example.

The leads of the present subject matter are easy to implant and deliver uncomplicated waveforms. Moreover, the baroreceptor pacing response is immediate and reversible.

As noted, further embodiments can include a sensor to monitor blood pressure. The sensor can sense a physiological parameter regarding an efficacy of the baroreflex therapy and provides a signal indicative of the efficacy of the baroreflex therapy. For example, a controller can be connected to a pulse generator to control the baroreflex stimulation signal and to the sensor to receive the signal indicative of the efficacy of the baroreflex therapy. In some examples, the pulse generator can be further adapted to generate a cardiac pacing signal, and the lead can include a second electrode to be positioned to deliver the cardiac pacing signal to capture the heart. Embodiments of the present electrodes allows for chronically indwelling, is easy to implant, and is safe and reliable.

In various embodiments of baroreceptor pacing according to the present subject matter, the system can deliver the pulse train intermittently with no sensing, or the system can be activated by the user when the user is at rest, or it can be activated by a timer to be periodically turned off and on, or it can be activated when the user goes to sleep, for example.

According to various embodiments, the stimulator circuitry and sensing circuitry of the present system can include one or more functions as described in the commonly assigned U.S. patent application filed on the same date as the present application : "Baroreflex Stimulation System to Reduce Hypertension," Ser. No. 10/746,134, filed on Dec 24, 2003 (Attorney Docket No. 279.675US1).

For example, various embodiments of the present subject matter relate to baroreflex stimulator systems. Such baroreflex stimulation systems are also referred to herein as neural stimulator (NS) devices or components. Examples of neural stimulators include anti-hypertension (AHT) devices or AHT components that are used to treat hypertension. Various embodiments of the present subject matter include stand-alone implantable baroreceptor stimulator systems, include implantable devices that have integrated NS and cardiac rhythm management (CRM) components, and include systems with at least one implantable NS device and an implantable CRM device capable of communicating with each other either wirelessly or through a wire lead connecting the implantable devices. Integrating NS and CRM functions that are either performed in the same or separate devices improves aspects of the NS therapy and cardiac therapy by allowing these therapies to work together intelligently.

Thus, various embodiments of the present subject matter provide an implantable NS device that automatically modulates baroreceptor stimulation using an IPS. Integrating the pressure sensor into the lead provides localized feedback for the stimulation. This localized sensing improves feedback control. For example, the integrated sensor can be used to compensate for inertia of the baroreflex such that the target is not continuously overshot. According to various embodiments, the device monitors pressure parameters such as mean arterial pressure, systolic pressure, diastolic pressure and the like. As mean arterial pressure increases or remains above a programmable target pressure, for example, the device stimulates baroreceptors at an increased rate to reduce blood pressure and control hypertension.

As mean arterial pressure decreases towards the target pressure, the device responds by reducing baroreceptor stimulation. In various embodiments, the algorithm takes into account the current metabolic state (cardiac demand) and adjusts neural stimulation accordingly. A NS device having an IPS is able to automatically modulate baroreceptor stimulation, which allows an implantable NS device to determine the level of hypertension in the patient and respond by delivering the appropriate level of therapy. However, it is noted that other sensors, including sensors that do not reside in an NS or neural stimulator device, can be used to provide close loop feedback control.

An aspect of the present subject matter relates to a chronically-implanted stimulation system specially designed to treat hypertension by monitoring blood pressure and stimulating baroreceptors to activate the baroreceptor reflex and inhibit sympathetic discharge from the vasomotor center. Baroreceptors are located in various anatomical locations such as the carotid sinus and the aortic arch. Other baroreceptor locations include the pulmonary artery, including the ligamentum arteriosum, and sites in the atrial and ventricular chambers. In various embodiments, the system is integrated into a pacemaker/defibrillator or other electrical stimulator system. Components of the system include a high-frequency pulse generator, sensors to monitor blood pressure or other pertinent physiological parameters, leads to apply electrical stimulation to baroreceptors, algorithms to determine the appropriate time to administer stimulation, and algorithms to manipulate data for display and patient management.

Various embodiments relates to a system that seeks to deliver electrically mediated NS therapy, such as AHT therapy, to patients. Various embodiments combine a "stand-alone" pulse generator with a minimally invasive, unipolar lead that directly stimulates baroreceptors in the vicinity of the heart, such as in the pulmonary artery. This embodiment is such that general medical practitioners lacking the skills of specialist can implant it. Various embodiments incorporate a simple implanted system that can sense parameters indicative of blood pressure. This system adjusts the therapeutic output (waveform amplitude, frequency, etc.) so as to maintain a desired quality of life. In various embodiments, an implanted system includes a pulse generating device and lead system, the stimulating electrode of which is positioned near endocardial baroreceptor tissues using transvenous implant technique(s).

Another embodiment includes a system that combines NS therapy with traditional bradyarrhythmia, tachyarrhythmia, and/or congestive heart failure (CHF) therapies. Some embodiments use an additional "baroreceptor lead" that emerges from the device header and is paced from a modified traditional pulse generating system. In another embodiment, a traditional CRM lead is modified to incorporate proximal electrodes that are naturally positioned near baroreceptor sites. With these leads, distal electrodes provide CRM therapy and proximal electrodes stimulate baroreceptors.

For example, Figure 15 schematically shows an unbiased lead 1900 having an electrode 1910 on a distal end and an active or positive fixation mechanism, such as a helix 1920. Electrode 1910 is intended to be implanted at or near a site of high baroreceptor concentration, such as baroreceptor site 206B in the pulmonary artery 204. At implant, lead 1900 would be passed through the heart and into the pulmonary artery. With the distal end of lead 1900 generally straight (unbiased), the act of implantation would naturally position the distal electrode end 1910 at or near baroreceptor site 206B. Stimulation at the 206B location by a single electrode can be preferable, in some instances, to advancing a longer lead device deeper into the pulmonary artery to attempt stimulation of a number of diffuse locations.

Various embodiments of the present subject matter relate to a method of sensing atrial activation and confining pulmonary artery stimulation to the atrial refractory period, preventing unintentional stimulation of nearby atrial tissue. An implantable baroreceptor stimulation device monitors atrial activation with an atrial sensing lead. A lead in the pulmonary artery stimulates baroreceptors in the vessel wall. However, instead of stimulating these baroreceptors continuously, the stimulation of baroreceptors in the pulmonary artery occurs during the atrial refractory period to avoid capturing nearby atrial myocardium, maintaining the intrinsic atrial rate and activation. Various embodiments of the present subject matter combine an implantable device for stimulating baroreceptors in the wall of the pulmonary artery with the capability for atrial sensing. Various embodiments stimulate baroreceptors in the cardiac fat pads, in the heart chambers, and/or afferent nerves.

In some embodiments, the pulse generator can include a transceiver and associated circuitry for use to communicate with a programmer or another external or internal device. Various embodiments have wireless communication capabilities. For example, some transceiver embodiments use a telemetry coil to wirelessly communicate with a programmer or another external or internal device.

The above description is intended to be illustrative, and not restrictive. Many other embodiments will be apparent to those of skill in the art upon reviewing the above description. The scope of the invention should, therefore, be determined with reference to the appended claims.

## Claims

1. An apparatus comprising:
a flexible lead body (110) extending from a proximal end to a distal end;
an electrode (130) coupled to the lead body;
an implantable pulse generator (140) electrically coupled to the electrode, the implantable pulse generator being adapted to deliver a baroreflex stimulation signal to a baroreceptor in the pulmonary artery via the electrode;
means for passively fixating the electrode within the pulmonary artery.

2. The apparatus of claim 1, wherein the means for passively fixating includes an expandable stent structure coupled to the lead.

3. The apparatus of claim 2, wherein the stent structure includes an expanded diameter dimensioned to abut a wall of a pulmonary artery.

4. The apparatus of claim 3, wherein the expanded diameter is about 10 to 20 mm.

5. The apparatus of claim 2, wherein the expandable stent expands to fix the lead in place by frictional forces.

6. The apparatus of claim 2, wherein the expandable stent structure includes a length of at least about 1 cm.

7. The apparatus of claim 1, wherein the means for passively fixating includes a biased portion proximate the distal end of the lead, the biased portion having an outer diameter dimensioned to abut a wall of a pulmonary artery.

8. An apparatus comprising:
a flexible lead body (110) extending from a proximal end to a distal end;
an expandable electrode (130) coupled proximate the distal end, the expandable electrode having an expanded diameter dimensioned to abut a wall of a pulmonary artery; and
an implantable pulse generator (140) electrically coupled to the expandable electrode, wherein the implantable pulse generator is adapted to deliver a baroreflex stimulation signal to a baroreceptor in the pulmonary artery via the electrode.

9. The apparatus of claim 8, wherein the expandable electrode includes a mesh surface.

10. The apparatus of claim 8, wherein the expandable electrode expands to fix the lead in place by frictional forces.

11. The apparatus of claim 8, wherein the expandable electrode includes a length of at least about 1 cm.

12. The apparatus of claim 8, wherein the expandable electrode includes an expanded diameter of about 10 to 20 mm.

13. The apparatus of claim 8, wherein the expandable electrode includes at least a partially electrically insulated surface.

14. The apparatus of claim 8, wherein the lead includes a second electrode located proximally from the expandable electrode.

15. The apparatus of claim 8, wherein the lead further includes a sensor to monitor blood pressure.

16. The apparatus of claim 8, wherein the electrode is adapted to be located near the ligamentum arteriosum of the left pulmonary artery.

17. The apparatus of claim 8, further including a sensor to sense a physiological parameter regarding an efficacy of the baroreflex therapy and to provide a signal indicative of the efficacy of the baroreflex therapy.

18. The apparatus of claim 17, further including a controller connected to the pulse generator to control the baroreflex stimulation signal and to the sensor to receive the signal indicative of the efficacy of the baroreflex therapy.

19. An apparatus comprising:
a flexible lead body (110) extending from a proximal end to a distal end, the distal end having a biased portion having an outer diameter dimensioned to abut a wall of a pulmonary artery;
an electrode (130) coupled proximate the distal end; and
an implantable pulse generator (140) electrically coupled to the electrode, wherein the implantable pulse generator is adapted to deliver a baroreflex stimulation signal to a baroreceptor in the pulmonary artery via the electrode.

20. The apparatus of claim 19, wherein the biased portion has an outer diameter between about 10 mm and about 20 mm.

21. The apparatus of claim 19, wherein the biased portion is adapted to fix the lead in place by frictional forces.

22. The apparatus of claim 19, wherein the biased portion includes a spiral configuration.

23. The apparatus of claim 19, wherein the electrode includes a porous surface.

24. The apparatus of claim 19, wherein the lead includes two or more discrete electrodes located on the biased portion of the lead.

25. The apparatus of claim 19, wherein the lead includes a second electrode located proximally from the distal end.

26. The apparatus of claim 19, further including a sensor to sense a physiological parameter regarding an efficacy of the baroreflex therapy and to provide a signal indicative of the efficacy of the baroreflex therapy.

27. The apparatus of claim 26, further including a controller connected to the pulse generator to control the baroreflex stimulation signal and to the sensor to receive the signal indicative of the efficacy of the baroreflex therapy.

28. The apparatus of claim 19, wherein the biased portion of the distal end is adapted to passively fixate the electrode within the pulmonary artery.

29. The apparatus of claim 19, wherein the biased portion includes a circular structure.

## Patentansprüche

1. Vorrichtung mit:
einem flexiblen Leitungskörper (110), der sich von einem proximalen Ende zu einem distalen Ende erstreckt;
einer Elektrode (130), die mit dem Leitungskörper gekoppelt ist;
einem implantierbaren Impulsgenerator (140), der mit der Elektrode elektrisch gekoppelt ist, wobei der implantierbare Impulsgenerator ausgebildet ist, ein Baroreflex-Stimulationssignal zu einem Barorezeptor in der Pulmonalarterie über die Elektrode abzugeben;
einer Einrichtung zum passiven Fixieren der Elektrode in der Pulmonalarterie.

2. Vorrichtung nach Anspruch 1, wobei die Einrichtung zum passiven Fixieren eine expandierbare Stentstruktur aufweist, die mit der Leitung gekoppelt ist.

3. Vorrichtung nach Anspruch 2, wobei die Stentstruktur einen expandierten Durchmesser aufweist, der so bemessen ist, daß er an einer Wand einer Pulmonalarterie anliegt.

4. Vorrichtung nach Anspruch 3, wobei der expandierte Durchmesser etwa 10 bis 20 mm beträgt.

5. Vorrichtung nach Anspruch 2, wobei der expandierbare Stent expandiert, um die Leitung durch Reibkräfte an Ort und Stelle zu fixieren.

6. Vorrichtung nach Anspruch 2, wobei die expandierbare Stentstruktur eine Länge von mindestens etwa 1 cm aufweist.

7. Vorrichtung nach Anspruch 1, wobei die Einrichtung zum passiven Fixieren einen vorgespannten Abschnitt nahe dem distalen Ende der Leitung aufweist, wobei der vorgespannte Abschnitt einen Außendurchmesser hat, der so bemessen ist, daß er an einer Wand einer Pulmonalarterie anliegt.

8. Vorrichtung mit:
einem flexiblen Leitungskörper (110), der sich von einem proximalen Ende zu einem distalen Ende erstreckt;
einer expandierbaren Elektrode (130), die nahe dem distalen Ende gekoppelt ist, wobei die expandierbare Elektrode einen expandierten Durchmesser hat, der so bemessen ist, daß er an einer Wand einer Pulmonalarterie anliegt; und
einem implantierbaren Impulsgenerator (140), der mit der expandierbaren Elektrode elektrisch gekoppelt ist, wobei der implantierbare Impulsgenerator ausgebildet ist, ein Baroreflex-Stimulationssignal zu einem Barorezeptor in der Pulmonalarterie über die Elektrode abzugeben.

9. Vorrichtung nach Anspruch 8, wobei die expandierbare Elektrode eine Maschenoberfläche aufweist.

10. Vorrichtung nach Anspruch 8, wobei die expandierbare Elektrode expandiert, um die Leitung durch Reibkräfte an Ort und Stelle zu fixieren.

11. Vorrichtung nach Anspruch 8, wobei die expandierbare Elektrode eine Länge von mindestens etwa 1 cm aufweist.

12. Vorrichtung nach Anspruch 8, wobei die expandierbare Elektrode einen expandierten Durchmesser von etwa 10 bis 20 mm aufweist.

13. Vorrichtung nach Anspruch 8, wobei die expandierbare Elektrode mindestens eine teilweise elektrisch isolierte Oberfläche aufweist.

14. Vorrichtung nach Anspruch 8, wobei die Leitung eine zweite Elektrode aufweist, die proximal von der expandierbaren Elektrode liegt.

15. Vorrichtung nach Anspruch 8, wobei die Leitung ferner einen Sensor aufweist, um den Blutdruck zu überwachen.

16. Vorrichtung nach Anspruch 8, wobei die Elektrode ausgebildet ist, nahe dem Ligamentum arteriosum der linken Pulmonalarterie zu liegen.

17. Vorrichtung nach Anspruch 8, ferner mit einem Sensor, um einen physiologischen Parameter bezüglich einer Wirksamkeit der Baroreflextherapie zu erfassen und ein Signal als Anzeige der Wirksamkeit der Baroreflextherapie zu liefern.

18. Vorrichtung nach Anspruch 17, ferner mit einer Steuerung, die verbunden ist mit dem Impulsgenerator, um das Baroreflex-Stimulationssignal zu steuern, und mit dem Sensor, um das Signal als Anzeige der Wirksamkeit der Baroreflextherapie zu empfangen.

19. Vorrichtung mit:
einem flexiblen Leitungskörper (110), der sich von einem proximalen Ende zu einem distalen Ende erstreckt, wobei das distale Ende einen vorgespannten Abschnitt mit einem Außendurchmesser hat, der so bemessen ist, daß er an einer Wand einer Pulmonalarterie anliegt;
einer Elektrode (130), die nahe dem distalen Ende gekoppelt ist; und
einem implantierbaren Impulsgenerator (140), der mit der Elektrode elektrisch gekoppelt ist, wobei der implantierbare Impulsgenerator ausgebildet ist, ein Baroreflex-Stimulationssignal zu einem Barorezeptor in der Pulmonalarterie über die Elektrode abzugeben.

20. Vorrichtung nach Anspruch 19, wobei der vorgespannte Abschnitt einen Außendurchmesser zwischen etwa 10 mm und etwa 20 mm hat.

21. Vorrichtung nach Anspruch 19, wobei der vorgespannte Abschnitt ausgebildet ist, die Leitung durch Reibkräfte an Ort und Stelle zu fixieren.

22. Vorrichtung nach Anspruch 19, wobei der vorgespannte Abschnitt eine Spiralkonfiguration aufweist.

23. Vorrichtung nach Anspruch 19, wobei die Elektrode eine poröse Oberfläche aufweist.

24. Vorrichtung nach Anspruch 19, wobei die Leitung zwei oder mehr diskrete Elektroden aufweist, die am vorgespannten Abschnitt der Leitung liegen.

25. Vorrichtung nach Anspruch 19, wobei die Leitung eine zweite Elektrode aufweist, die proximal vom distalen Ende liegt.

26. Vorrichtung nach Anspruch 19, ferner mit einem Sensor, um einen physiologischen Parameter bezüglich einer Wirksamkeit der Baroreflextherapie zu erfassen und ein Signal als Anzeige der Wirksamkeit der Baroreflextherapie zu liefern.

27. Vorrichtung nach Anspruch 26, ferner mit einer Steuerung, die verbunden ist mit dem Impulsgenerator, um das Baroreflex-Stimulationssignal zu steuern, und mit dem Sensor, um das Signal als Anzeige der Wirksamkeit der Baroreflextherapie zu empfangen.

28. Vorrichtung nach Anspruch 19, wobei der vorgespannte Abschnitt des distalen Endes ausgebildet ist, die Elektrode in der Pulmonalarterie passiv zu fixieren.

29. Vorrichtung nach Anspruch 19, wobei der vorgespannte Abschnitt eine kreisförmige Struktur aufweist.

## Revendications

1. Appareil, comprenant :
un corps de cordon flexible (110) s'étendant d'une extrémité proximale à une extrémité distale ;
une électrode (130) couplée au corps de cordon ;
un générateur d'impulsions implantable (140) connecté électriquement à l'électrode, le générateur d'impulsions implantable étant conçu pour fournir un signal de stimulation de baroréflexe à un barorécepteur dans l'artère pulmonaire par l'intermédiaire de l'électrode ;
des moyens de fixation passive de l'électrode à l'intérieur de l'artère pulmonaire.

2. Appareil selon la revendication 1, dans lequel les moyens de fixation passive comprennent une structure de stent dilatable couplée au cordon.

3. Appareil selon la revendication 2, dans lequel la structure de stent présente un diamètre à l'état dilaté dimensionné de sorte à buter contre une paroi d'une artère pulmonaire.

4. Appareil selon la revendication 3, dans lequel le diamètre à l'état dilaté est compris sensiblement entre 10 et 20 mm.

5. Appareil selon la revendication 2, dans lequel le stent dilatable se dilate pour fixer le cordon en place au moyen de forces de friction.

6. Appareil selon la revendication 2, dans lequel la structure le stent dilatable présente une longueur d'au moins environ 1 cm.

7. Appareil selon la revendication 1, dans lequel les moyens de fixation passive comprennent une partie précontrainte à proximité de l'extrémité distale du cordon, la partie précontrainte ayant un diamètre externe dimensionné de sorte à buter contre une paroi d'une artère pulmonaire.

8. Appareil, comprenant :
un corps de cordon flexible (110) s'étendant d'une extrémité proximale à une extrémité distale ;
une électrode dilatable (130) couplée à proximité de l'extrémité distale, l'électrode dilatable ayant un diamètre à l'état dilaté dimensionné de sorte à buter contre une paroi d'une artère pulmonaire ; et
un générateur d'impulsions implantable (140) connecté électriquement à l'électrode dilatable, dans lequel le générateur d'impulsions implantable est conçu pour fournir un signal de stimulation de baroréflexe à un barorécepteur dans l'artère pulmonaire par l'intermédiaire de l'électrode.

9. Appareil selon la revendication 8, dans lequel l'électrode dilatable comprend une surface maillée.

10. Appareil selon la revendication 8, dans lequel l'électrode dilatable se dilate pour fixer le cordon en place au moyen de forces de friction.

11. Appareil selon la revendication 8, dans lequel l'électrode dilatable présente une longueur d'au moins environ 1 cm.

12. Appareil selon la revendication 8, dans lequel l'électrode dilatable présente un diamètre à l'état dilaté compris entre sensiblement 10 et 20 mm.

13. Appareil selon la revendication 8, dans lequel l'électrode dilatable comprend au moins une surface à isolation électrique partielle.

14. Appareil selon la revendication 8, dans lequel le cordon comprend une seconde électrode située proximalement de l'électrode dilatable.

15. Appareil selon la revendication 8, dans lequel le cordon comprend en outre un capteur pour surveiller la pression artérielle.

16. Appareil selon la revendication 8, dans lequel l'électrode est adaptée pour être positionnée à proximité du ligament artériel de l'artère pulmonaire gauche.

17. Appareil selon la revendication 8, comprenant en outre un capteur pour capter un paramètre physiologique concernant une efficacité de la thérapie du baroréflexe et pour fournir un signal indiquant l'efficacité de la thérapie du baroréflexe.

18. Appareil selon la revendication 17, comprenant en outre un contrôleur relié au générateur d'impulsions pour contrôler le signal de stimulation du baroréflexe, et au capteur pour recevoir le signal indiquant l'efficacité de la thérapie du baroréflexe.

19. Appareil, comprenant :
un corps de cordon flexible (110) s'étendant d'une extrémité proximale à une extrémité distale, l'extrémité distale ayant une partie précontrainte présentant un diamètre externe dimensionné de sorte à buter contre une paroi d'une artère pulmonaire ;
une électrode (130) couplée à proximité de l'extrémité distale ; et
un générateur d'impulsions implantable (140) connecté électriquement à l'électrode, le générateur d'impulsions implantable étant adapté pour fournir un signal de stimulation de baroréflexe à un barorécepteur dans l'artère pulmonaire par l'intermédiaire de l'électrode.

20. Appareil selon la revendication 19, dans lequel la partie précontrainte a un diamètre externe allant d'environ 10 mm à environ 20 mm.

21. Appareil selon la revendication 19, dans lequel la partie précontrainte est adaptée pour fixer le cordon en place au moyen de forces de friction.

22. Appareil selon la revendication 19, dans lequel la partie précontrainte présente une configuration hélicoïdale.

23. Appareil selon la revendication 19, dans lequel l'électrode comprend une surface poreuse.

24. Appareil selon la revendication 19, dans lequel le cordon comprend deux ou plusieurs électrodes discrètes disposées sur la partie précontrainte du cordon.

25. Appareil selon la revendication 19, dans lequel le cordon comprend une seconde électrode située proximalement de l'extrémité distale.

26. Appareil selon la revendication 19, comprenant en outre un capteur pour capter un paramètre physiologique concernant une efficacité de la thérapie du baroréflexe et pour fournir un signal indiquant l'efficacité de la thérapie du baroréflexe.

27. Appareil selon la revendication 26, comprenant en outre un organe de commande relié au générateur d'impulsions pour commander le signal de stimulation du baroréflexe, et au capteur pour recevoir le signal indiquant l'efficacité de la thérapie du baroréflexe.

28. Appareil selon la revendication 19, dans lequel la partie précontrainte de l'extrémité distale est adaptée pour fixer passivement l'électrode à l'intérieur de l'artère pulmonaire.

29. Appareil selon la revendication 19, dans lequel la partie précontrainte comprend une structure circulaire.
